(19) 

Europäisches
Patentamt
European
Patent Office
Office européen
des brevets

(11)  **EP 4 297 828 B1**

(12)  **EUROPEAN PATENT SPECIFICATION**

(45) Date of publication and mention
of the grant of the patent:
**13.05.2026  Bulletin 2026/20**

(21) Application number: **22709907.4**

(22) Date of filing: **24.02.2022**

(51) International Patent Classification (IPC):
*A61M 5/28* (2006.01)   *A61J 1/14* (2023.01)
*A61J 1/20* (2006.01)   *B65B 3/04* (2006.01)
*A61M 5/31* (2006.01)   *A61M 5/178* (2006.01)
*B65B 3/00* (2006.01)   *A61J 1/06* (2006.01)
*B65B 3/14* (2006.01)   *B65B 3/26* (2006.01)
*B65B 31/00* (2006.01)

(52) Cooperative Patent Classification (CPC):
**A61M 5/28; A61J 1/2096; B65B 3/003; B65B 3/14;
B65B 3/26; B65B 31/00;** A61J 1/062; A61J 1/065;
A61J 1/2013; A61M 5/1782; A61M 2005/3114;
A61M 2209/045

(86) International application number:
**PCT/US2022/017680**

(87) International publication number:
**WO 2022/182858 (01.09.2022 Gazette 2022/35)**

(54) **SELF-ASPIRATING SYRINGE SYSTEMS, CARTRIDGES, AND METHODS**

SELBSTASPIRATIONSSPRITZENSYSTEME, KARTUSCHEN UND VERFAHREN

SYSTÈMES DE SERINGUE AUTO-ASPIRANTE, CARTOUCHES ET MÉTHODES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **25.02.2021   US 202163153420 P**

(43) Date of publication of application:
**03.01.2024   Bulletin 2024/01**

(73) Proprietor: **Eli Lilly and Company
Indianapolis, IN 46285 (US)**

(72) Inventor: **CHELLQUIST, Eric Magnus
Indianapolis, Indiana 46206-6288 (US)**

(74) Representative: **J A Kemp LLP
80 Turnmill Street
London EC1M 5QU (GB)**

(56) References cited:
**EP-B1- 1 825 878     US-A1- 2015 013 827**

EP 4 297 828 B1

## Description

### CROSS-REFERENCE TO RELATED APPLICATION

This application claims the benefit of U.S. Provisional Application No. 63/153,420, filed February 25, 2021.

### BACKGROUND

The present disclosure relates to syringe systems for delivering injectable drugs to subjects, and, in particular, self-aspirating syringe systems and cartridges having reduced-pressure volumes that facilitate self-aspiration.

Injectable drugs are commonly provided in multiple-dose or single-dose vials, and such agents are dispensed from the vials to hypodermic syringes for subsequent delivery to patients. More specifically, this process typically begins by holding the syringe with the needle pointing upwardly. The syringe's plunger is then retracted to fill the syringe with air, specifically a volume of air equal to the volume of the injectable drug to be administered to a patient. The needle of the syringe is then inserted through a stopper coupled to the opening of a vial, and the plunger is advanced to deliver the air from the syringe to the vial. Next, the vial and the syringe are inverted, and the needle's tip is held in the injectable drug. The plunger is then retracted to fill the syringe with the injectable drug, and the needle is removed from the vial. The syringe is then ready to deliver the injectable drug to a patient.

For some individuals, such as diabetics, the above process is relatively difficult or inconvenient. In other settings, such as high-throughput contract pharmacies, the above process is overly time consuming. Accordingly, it would be desirable to provide improved syringe systems.

EP1825878A1 discloses an assembly for administering to a patient a dose of active material, e.g. a radio active substance containing fluid and/or a contrast medium fluid for imaging purposes. The assembly comprises a container, having a ejection side with an exit and a control side; and a plunger movable from the proximal to the distal side of the container to eject the dose, which is pre-filled in the container, from the exit. Further, the assembly comprises a conduit configuration, which is connectable to the exit of the container and to the patient. The plunger in the assembly comprises a passage for a flushing fluid, which passage is normally closed and selectively opened. For selectively opening the passage the assembly comprises an opener, which is arrangable at the exit to act on the plunger to open the passage with the plunger moved to a position in proximity of the exit.

US2015013827A1 discloses a reservoir for a drug delivery system is provided, wherein the reservoir comprises a cavity, the cavity being filled with a dose of a fluid drug such that the dose of the drug occupies 95% or more of the cavity, wherein the dose volume is less than 5 ml. Furthermore, a method for filling a reservoir for a drug delivery system is provided.

### SUMMARY

The scope of the invention is defined in the appended claims.

According to an embodiment of the present disclosure, a cartridge is configured to receive an injectable drug. The cartridge includes a body, and the body includes a proximal end portion having a proximal opening, a distal end portion having a distal opening, and a bore coupling the proximal opening and the distal opening. The cartridge further includes a pierceable septum coupled to the distal opening and a plunger movably carried within the bore. A volume is disposed in the bore between the pierceable septum and the plunger. The volume has a reduced pressure, and the reduced pressure is less than atmospheric pressure. A stopper is detachably coupled to the distal opening.

According to another embodiment of the present disclosure, a syringe system is configured for delivering an injectable drug to a subject. The syringe system includes a cartridge, a vial, a transfer device, and a delivery needle. The cartridge includes a body having a bore, a plunger movably carried within the bore, and a volume disposed in the bore. The volume has a reduced pressure, and the reduced pressure is less than atmospheric pressure. The vial carries the injectable drug. The transfer device is configured to deliver the injectable drug from the vial to the bore of the cartridge. The delivery needle is configured to be coupled to the distal end portion of the cartridge and deliver the injectable drug from the cartridge to the subject.

According to yet another embodiment of the present disclosure, a method of manufacturing a cartridge, the cartridge being configured to receive an injectable drug, includes: providing a body including a distal opening, a proximal opening, and bore extending between the distal opening and the proximal opening; positioning a plunger in the bore; coupling a stopper to the proximal opening; reducing a pressure in the bore to less than atmospheric pressure; and sealing the distal opening.

According to yet another embodiment of the present disclosure, a method of delivering an injectable drug from an internal chamber of a vial to an internal bore of a cartridge, the internal bore initially having a reduced pressure volume, includes venting the internal chamber of the vial such that the injectable drug is exposed to atmospheric pressure; coupling a transfer device to the vial; coupling the transfer device to the cartridge, the transfer device thereby facilitating fluid communication between the internal chamber of the vial and the internal bore of the cartridge; and permitting the injectable drug to flow from the internal chamber of the vial, through the transfer device, and into the internal bore of the cartridge.

## BRIEF DESCRIPTION OF THE DRAWINGS

The above-mentioned and other advantages and objects of this disclosure, and the manner of attaining them, will become more apparent, and the disclosure itself will be better understood, by reference to the following description of embodiments of the disclosure taken in conjunction with the accompanying drawings, wherein:

FIG. 1 is a side sectional view of a cartridge for a syringe system according to an embodiment of the present disclosure.

FIG. 2 is a flow diagram illustrating a method of delivering an injectable drug to a subject according to an embodiment of the present disclosure.

FIG. 3 is a side sectional view of a vial carrying an injectable drug and the cartridge of FIG. 1; the vial and the cartridge are provided according to the method illustrated in FIG. 2.

FIG. 4 is a side sectional view of a transfer needle being coupled to the vial according to the method illustrated in FIG. 2.

FIG. 5 is a side sectional view of a needle hub being coupled to the transfer needle according to the method illustrated in FIG. 2.

FIG. 6 is a side sectional view of the cartridge being coupled to the needle hub, the transfer needle, and the vial according to the method illustrated in FIG. 2.

FIG. 7 is a side sectional view of an injectable drug flowing from the vial to the cartridge according to the method illustrated in FIG. 2.

FIG. 8 is a side sectional view of the vial, the transfer needle, a nut, and a stopper being uncoupled from the cartridge according to the method illustrated in FIG. 2.

FIG. 9 is a side sectional view of a delivery needle and an actuation rod being coupled to the cartridge according to the method illustrated in FIG. 2.

FIG. 10 is a side sectional view of the cartridge coupled to the vial via a flexible conduit according to an alternative of the method illustrated in FIG. 2.

FIG. 11 is a flow diagram illustrating a method of manufacturing a cartridge according to an embodiment of the present disclosure.

FIG. 12 is a side sectional view of a plunger being coupled to a body of the cartridge according to the method illustrated in FIG. 11.

FIG. 13 is a side sectional view of a stopper, a nut, and a pierceable septum being coupled to the body of the cartridge according to the method illustrated in FIG. 11.

FIG. 14 is a side sectional view of the cartridge positioned in a pressure reduction chamber according to the method illustrated in FIG. 11.

FIG. 15 is a side sectional view of the septum being coupled to the cartridge according to the method illustrated in FIG. 11.

FIG. 16 is a side sectional view of a seal being coupled to the cartridge according to the method illustrated in FIG. 11.

Corresponding reference characters indicate corresponding parts throughout the several views. Although the drawings represent embodiments of the present invention, the drawings are not necessarily to scale, and certain features may be exaggerated or omitted in some of the drawings in order to better illustrate and explain the present invention.

## DETAILED DESCRIPTION

Syringe systems according to the present disclosure dispense one or more injectable drugs, which may also be referred to as medications or therapeutic agents. Such injectable drugs may include, for example, vaccines, antibodies, insulins, insulin analogs, insulin derivatives, GLP-1 receptor agonists, epinephrine, anaesthetics, analgesics, steroids, imaging/contrast agents, radiopharmaceutical agents, or any other drug that is capable of delivery by systems according to the present disclosure. Syringe systems according to the present disclosure are operated in a manner generally as described herein by a user (for example, a healthcare professional, a caregiver, or another person) to deliver one or more injectable drugs to a subject (for example, a patient, the user himself or herself, another person, or an animal).

Any directional references used with respect to any of the Figures, such as right or left, up or down, or top or bottom, are intended for convenience of description, and does not limit the present disclosure or any of its components to any particular positional or spatial orientation.

Self-aspirating syringe systems, cartridges, and methods according to embodiments of the present disclosure provide one or more of various advantages. For example, systems and cartridges according to embodiments of the present disclosure may be relatively simple to operate and inexpensive to manufacture.

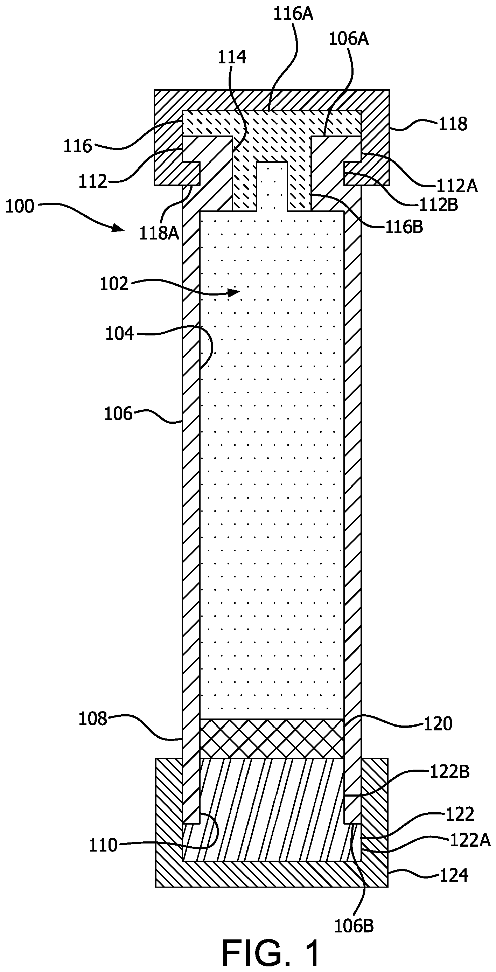

FIG. 1 illustrates a cartridge 100 for a syringe system (shown elsewhere) according to an embodiment of the present disclosure. The cartridge 100 is illustrated in an initial configuration, or a configuration in which the cartridge 100 is provided to a user prior to delivering an injectable drug to a subject. More specifically, the cartridge 100 is illustrated as having a reduced-pressure volume 102 within an internal bore 104 of a main body 106. The volume 102 has a reduced pressure relative to atmospheric pressure, and the reduced pressure facilitates aspirating an injectable drug from a vial (shown elsewhere) and delivering the injectable drug to the bore 104 of the cartridge 100. These aspects are described in further detail below.

The reduced-pressure volume 102 may comprise a gas (for example, air) or the reduced-pressure volume 102 may be a vacuum. The reduced-pressure volume 102 may have any of various pressures that are less than atmospheric pressure (that is, 1 atm (101.325 kPa)), including, for example, 0.9 atm (91.193 kPa), 0.8 atm (81.060 kPa), 0.7 atm (70.928 kPa), 0.6 atm (60.795 kPa), 0.5 atm (50.663 kPa), 0.4 atm (40.530 kPa), 0.3 atm (30.398 kPa), 0.2 atm (20.265 kPa), 0.1 atm (10.133 kPa), or 0.0 atm (0.0 kPa). As described in further detail below, the pressure of the reduced-pressure volume 102 affects the volume of injectable drug that is aspirated from a vial with the volume at a higher pressure than the reduced-pressure volume 102, which may be, for example, at atmoshpheric pressure, and transferred to the bore 104 of the cartridge 100.

With continued reference to FIG. 1, the cartridge 100 also includes various other components that facilitate initially sealing the bore 104 from the external environment, transferring an injectable drug to the cartridge 100, and delivering the injectable drug to a subject. More specifically, the body 106 of the cartridge 100 includes a proximal end portion 108 that has a proximal opening 110 and a distal end portion 112 that has a distal opening 114. The bore 104 couples and extends between the proximal opening 110 and the distal opening 114. At the distal end portion 112, a pierceable septum 116 is coupled to the distal opening 114, and a seal 118 covers the pierceable septum 116. The distal end portion 112 may be configured to receive a needle assembly. In one example, the distal end portion 112 defines an outer radial flange 112A and a reduced neck region 112B that is adjacent the flange 112A, such as shown in FIG. 1. At the proximal end portion 108, a plunger 120 is movably carried within the bore 104, and a vacuum stopper 122 is detachably coupled to the distal opening 114 such that the plunger 120 is disposed between the stopper 122 and the reduced-pressure volume 102. The proximal end portion 108 also includes a fastener, more specifically a vacuum nut 124, that secures the stopper 122 between the nut 124 and the plunger 120.

In one embodiment, as shown in FIG. 1, the pierceable septum 116 is constructed to include a distal head portion 116A that is disposed against the distal facing surface 106A defined by the body 106 and a proximal reduced-area portion 116B extending proximally from the distal head portion 116A and sized to engage the inner wall of the body 106 that defines the distal end opening 112. In one embodiment, as shown in FIG. 1, the seal 118 is constructed having a cup shape with an inner radial lip 118A proximally spaced from an inner axial wall of the seal 118, whereby the inner radial lip 118A is sized to extend within and fit within the reduced neck region 112B of the body's distal end portion 112, and the interior space of the seal 118 capturing the distal head portion 116A of the septum 116 and the thickness of the flange 112A. In other embodiments, the seal 118 threadably couples to the body 106. In one embodiment, as shown in FIG. 1, the stopper 122 is constructed to have a proximal head portion 122A that is disposed against the proximal facing surface 106B or edge defined by the body 106 and a distal reduced-area portion 122B extending distally from the proximal head portion 122A and sized to engage the inner wall defining the inner bore 104 at the proximal end opening 110. In one embodiment, as shown in FIG. 1, the nut 124 is constructed to have a cup shape having an inner diameter sized to fit over the outer diameter of the proximal head portion 122A of the stopper 122 and the outer diameter of the body 106.

The components of the cartridge 100 may be constructed of various appropriate materials. The body 106 may be constructed of, for example, glass or plastic. The pierceable septum 116, the plunger 120, the stopper 122, and/or the nut 124 may be constructed of, for example, plastic. The seal 118 may be constructed of, for example, metal.

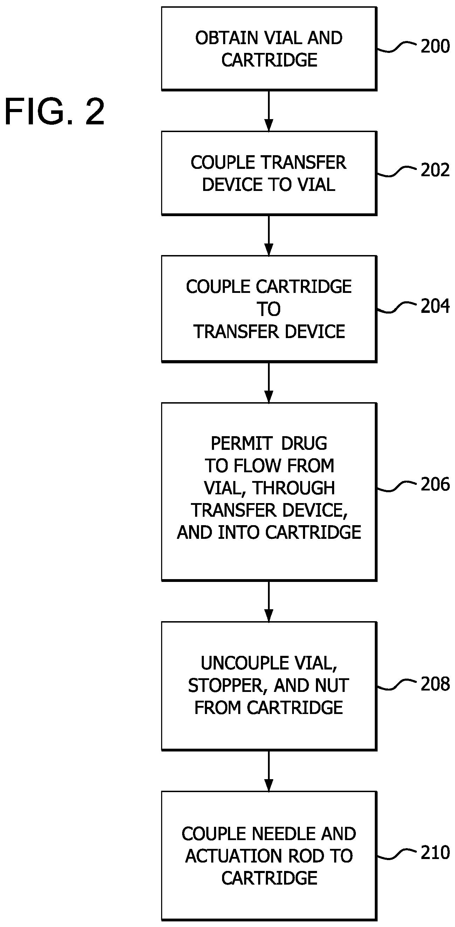

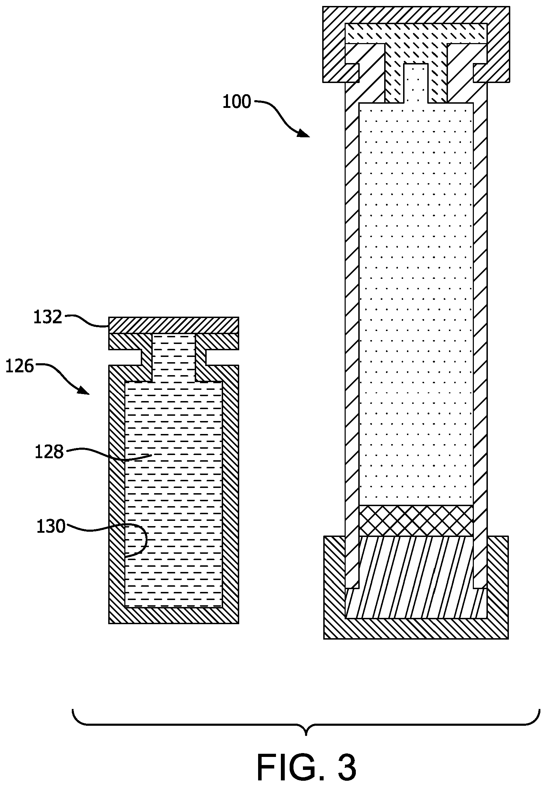

FIG. 2 is a flow diagram illustrating a method of using a syringe system to deliver an injectable drug to a subject, according to an embodiment of the present disclosure. FIGS. 3-10 illustrate various actions associated with the method.

The method begins, as shown at block 200 of FIG. 2 and as shown in FIG. 3, by providing a drug vial 126, or a vial 126 carrying an injectable drug 128 in an internal chamber 130, and the cartridge 100 in its initial configuration (that is, having the reduced-pressure volume 102 as shown in FIG. 1). The method continues by piercing a septum 132 of the vial 126 with a vent filter (not shown) such that the injectable drug is exposed to atmospheric pressure.

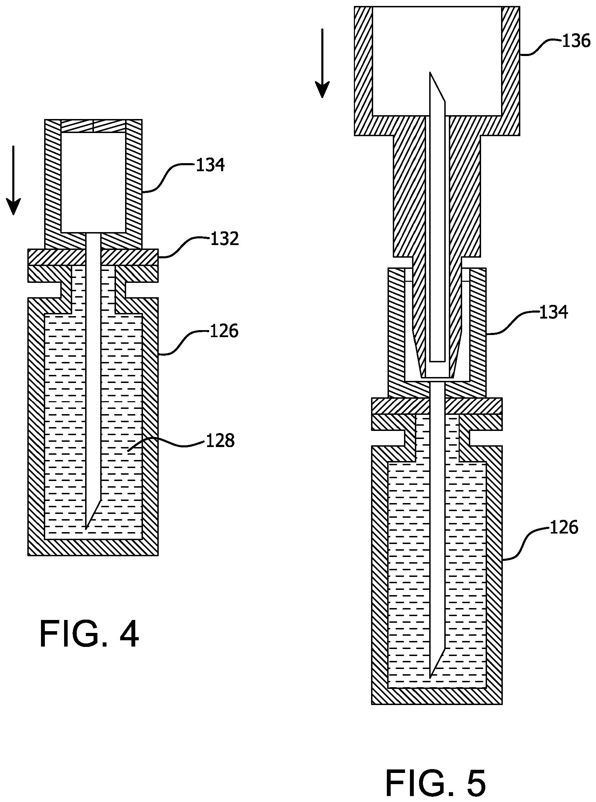

With the vent filter remaining coupled to the vial 126, the method continues, as shown at block 202, by coupling a transfer device to the vial 126. More specifically, and as shown in FIG. 4, the transfer device may include a transfer needle 134 that pierces the septum 132 of the vial 126 to access the injectable drug 128. As shown in FIG. 5, the transfer device may further include a needle hub 136 that couples to the transfer needle 134.

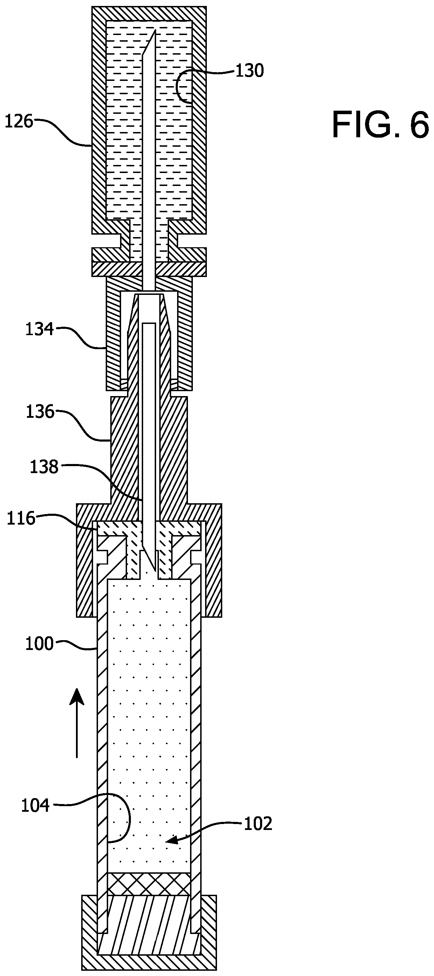

Next, after the seal 118 (shown elsewhere) is removed from the distal end portion 112 of the cartridge 100 and, as shown at block 204, the cartridge 100 is coupled to the transfer device and the vial 126. More specifically, and as shown in FIG. 6, the cartridge 100 is coupled to an end of the needle hub 136 opposite the transfer needle 134 and the vial 126. As a result, a transfer cannula 138 of the needle hub 136 pierces the septum 116, and the transfer cannula 138 thereby accesses the internal bore 104 and reduced-pressure volume 102 of the cartridge 100. As a result, the transfer device facilitates fluid communication between the internal chamber 130 of the vial 126 and the internal bore 104 of the cartridge 100.

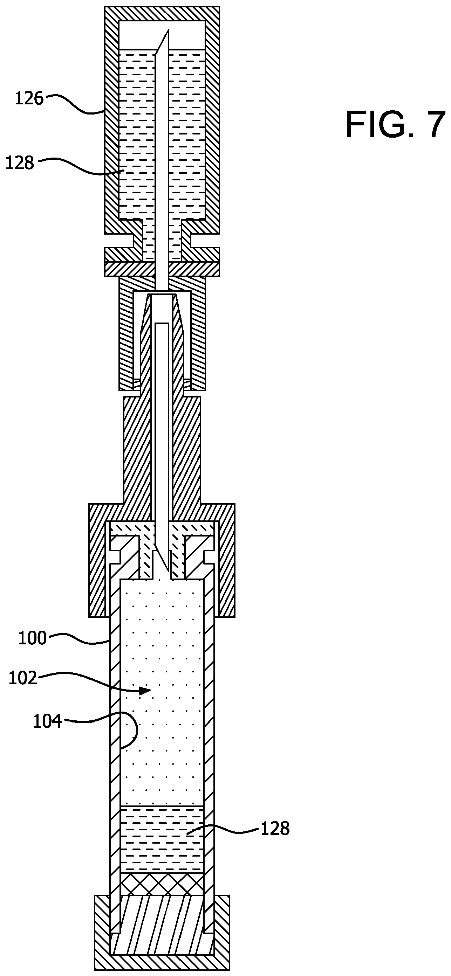

The method continues at block 206 by permitting the injectable drug 128 to flow from the internal chamber 130 of the vial 126, through the transfer device, and into the internal bore 104 of the cartridge 100. More specifically, and referring to FIG. 7, because the injectable drug 128 in the vial 126 is exposed to atmospheric pressure and the reduced-pressure volume 102 is present in the bore 104, the injectable drug 128 flows from the vial 126 to the cartridge 100. Flow of the injectable drug 128 terminates when any gas in the reduced-pressure volume 102 is sufficiently compressed and reaches atmospheric pressure or, if the reduced-pressure volume 102 is a vacuum, when the cartridge 100 is completely filled by the injectable drug 128.

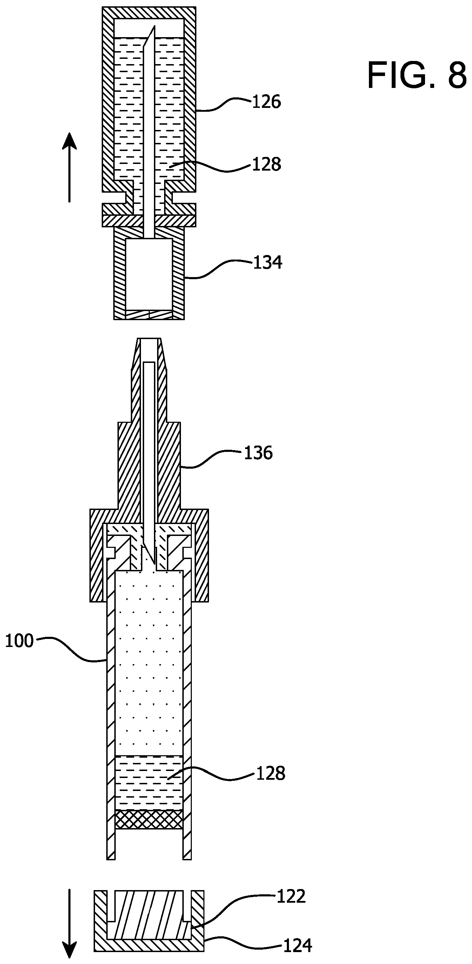

The method continues at block 208 by uncoupling the vial 126, the stopper 122, and the nut 124 from the cartridge 100. More specifically, and as shown in FIG. 8, the vial 126 and the transfer needle 134 are uncoupled from the needle hub 136. The vial 126 may be discarded if it carries a single dose of the injectable drug 128 or used with additional cartridges 100 if it carries multiple doses of the injectable drug 128.

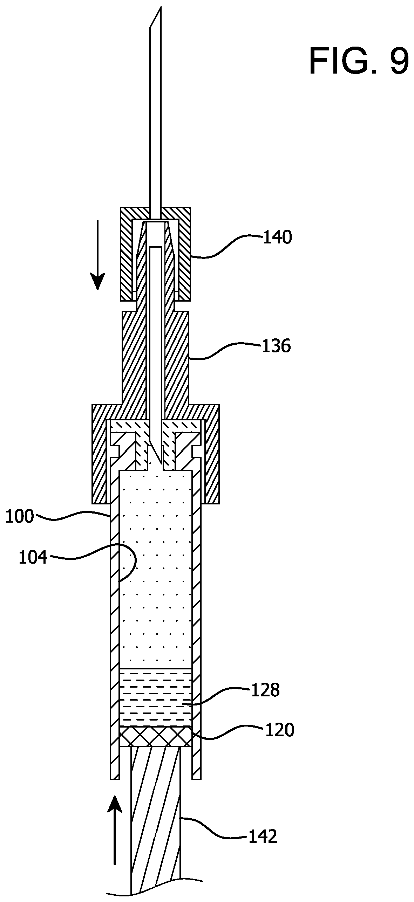

Next, and as shown at block 210 and in FIG. 9, a delivery needle 140 is coupled to the needle hub 136 and an actuation rod 142 is coupled to the plunger 120 (for example, via a threaded post carried by the plunger 120 - not shown). A user may advance the rod 142 and the plunger 120 to expel any gas remaining in the bore 104 of the cartridge 100, and the syringe system is then ready to deliver the injectable drug 128 to a subject.

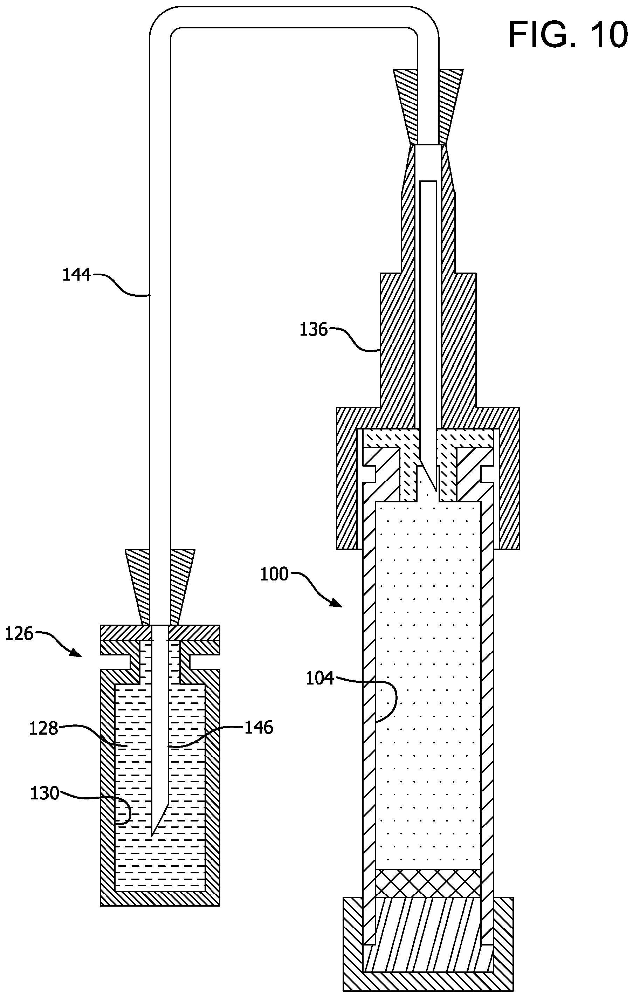

The method described above may be modified in various manners. For example, a different transfer device may be used to deliver the injectable drug 128 from the vial 126 to the cartridge 100. As a specific example and referring to FIG. 10, the transfer device may include tubing or a flexible conduit 144 that couples to a transfer needle 146 and the needle hub 136 and, as a result, facilitates fluid communication between the internal chamber 130 of the vial 126 and the internal bore 104 of the cartridge 100.

The cartridge 100 may alternatively be used in connection with other methods. For example, the cartridge 100 may be used to aspirate a liquid from a subject. More specifically, the cartridge 100 may be used to aspirate a liquid from the lung of a subject. In such a method, the cartridge 100 may be used with an actuation needle hub (not shown) to pierce the lung of the subject with the external needle of the hub. Subsequently, the internal needle of the hub may pierce the septum 116 of the cartridge 100 to facilitate aspirating the liquid into the cartridge 110.

As described briefly above, the initial pressure of the reduced-pressure volume 102 affects the volume of injectable drug that is aspirated from a vial and transferred to the bore 104 of the cartridge 100. More specifically, and based on the ideal gas law (if the temperature and amount of any gas in the reduced-pressure volume 102 remain constant), the amount of an injectable drug, $V_a$, transferred into the bore 104 of the cartridge 100 is as follows:

$$V_a = V_i \left(1 - (P_i/P_f)\right)$$

where:

$V_i$ is the initial volume of the reduced-pressure volume 102;
$P_i$ is the initial pressure of the reduced-pressure volume 102; and
$P_f$ is the final pressure of the reduced-pressure volume 102 (that is, after transferring the injectable drug to the bore 104), typically atmospheric pressure.

Some syringe systems according to embodiments of the present disclosure may include various cartridges having different sizes and/or reduced-pressure volumes with different pressures, and a user may select a cartridge appropriate for delivering a specific volume of an injectable drug to a subject.

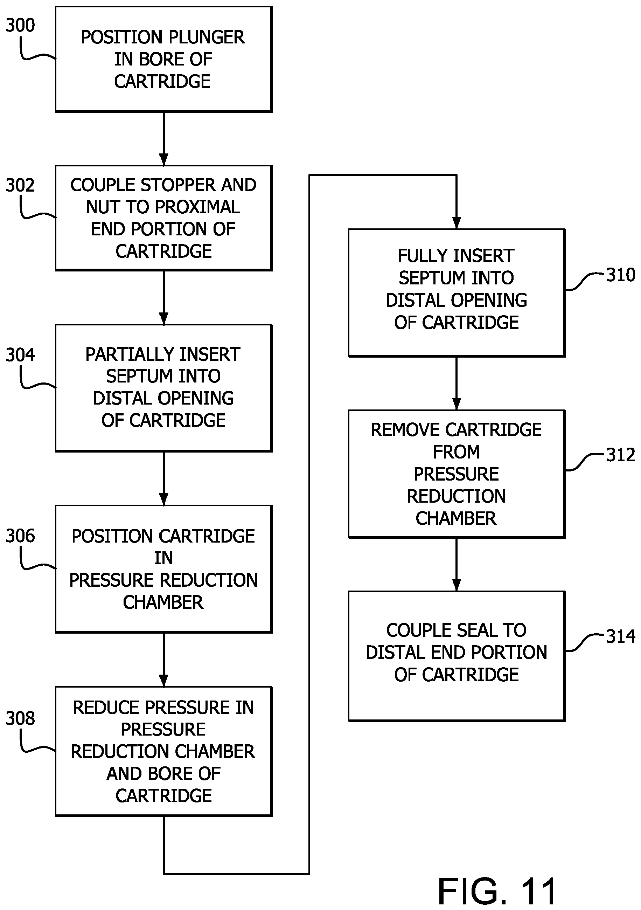

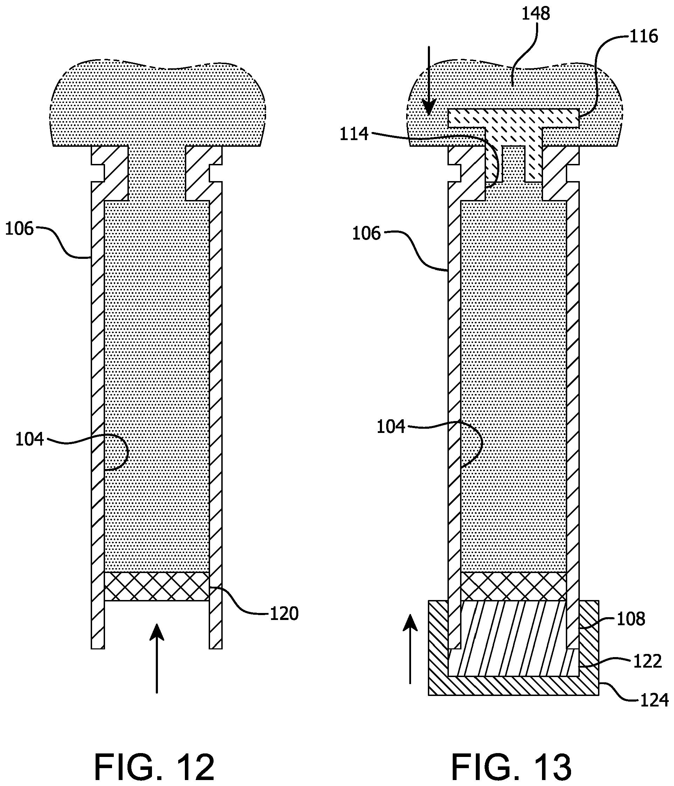

FIG. 11 is a flow diagram illustrating a method of manufacturing the cartridge 100 according to an embodiment of the present disclosure. FIGS. 12-16 illustrate various actions associated with the method.

The method begins as shown at block 300 by positioning the plunger 120 in bore 104 of the cartridge 100. More specifically, and as shown in FIG. 12, the plunger 120 is positioned in the bore 104 of the body 106 while the bore 104 is exposed to a gas, for example, air, at a non-reduced pressure, such as atmospheric pressure.

Next, and as shown at block 302 and in FIG. 13, the stopper 122 and the nut 124 are coupled to the proximal end portion 108 of the body 106. As shown at block 304 and in FIG. 13, the septum 116 is partially inserted in the distal opening 114 such that the bore 104 remains in fluid communication with the external environment 148 of the body 106.

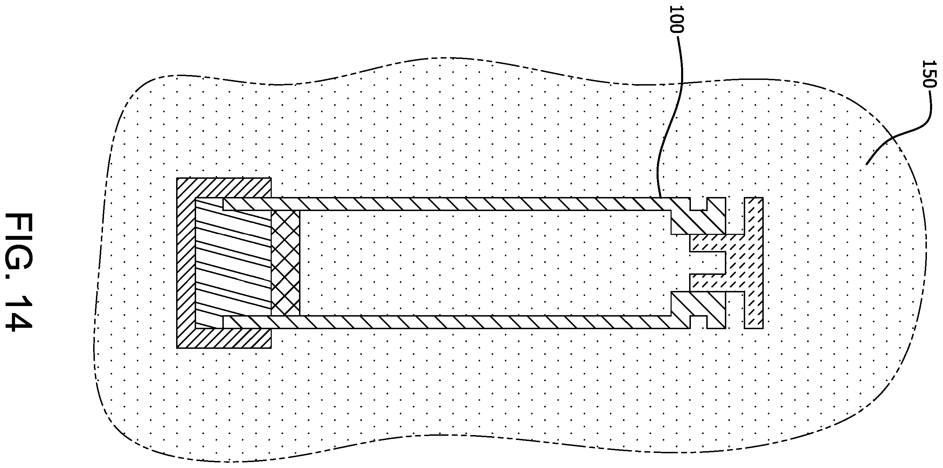

Next, and as shown at block 306 and in FIG. 14, the cartridge 100 is positioned in a vacuum or pressure reduction chamber 150. Then, and as shown at block 308, the pressure in the pressure reduction chamber 150 and the bore 104 of the cartridge 100 is reduced.

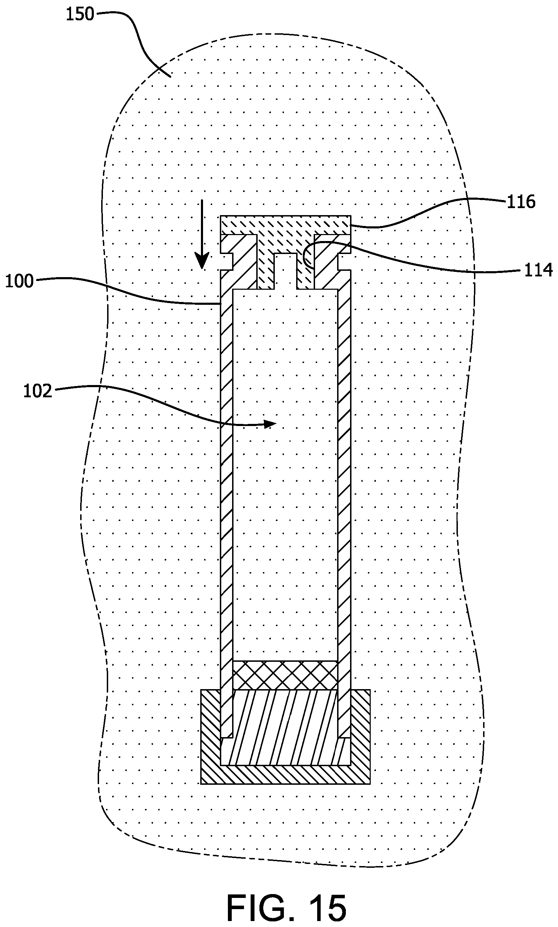

Next, and as shown at block 310 and in FIG. 15, the septum 116 is fully inserted into the distal opening 114 of the cartridge 100, thereby sealing the vacuum or reduced pressure of the vacuum or pressure reduction chamber 150 in the cartridge 100 as the reduced-pressure volume 102. Then, and as shown at block 312, the cartridge 100 is removed from the pressure reduction chamber 150.

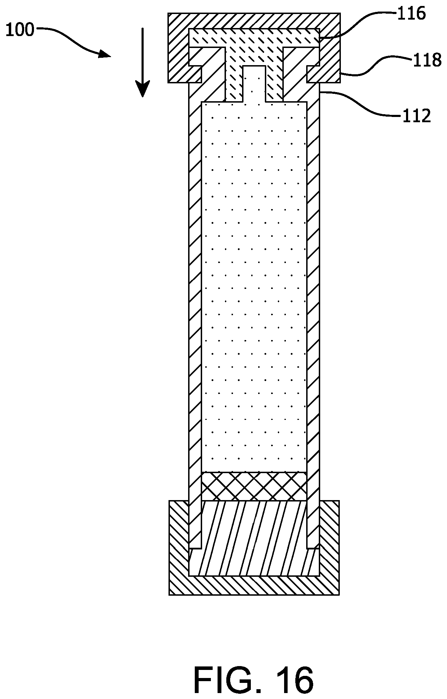

Finally, and as shown at block 314 and in FIG. 16, the seal 118 is coupled to the distal end portion 112 of the cartridge 100 and over the septum 116.

While this invention has been shown and described as having preferred designs, the present invention may be modified within the scope of this disclosure. This application is therefore intended to cover any variations, uses or adaptations of the invention that fall under the scope of the appended set of claims.

**Claims**

1. A cartridge (100) for receiving an injectable drug, comprising:

   a body (106) comprising:

   a proximal end portion (108) comprising a proximal opening (110);
   a distal end portion (112) comprising a distal opening (114);
   a bore (104) coupling the proximal opening (110) and the distal opening (114);

   a pierceable septum (116) coupled to the distal opening (114);
   a plunger (120) movably carried within the bore (104);
   a volume disposed in the bore (104) between the pierceable septum (116) and the plunger (120), the volume having a reduced pressure, the reduced pressure being less than atmospheric pressure;
   **characterised in that**
   a stopper (122) is detachably coupled to the proximal opening (110),
   wherein the plunger (120) is configured to couple to an actuating rod (142) upon detachment of the stopper (122) from the body (106).

2. The cartridge (100) of claim 0, wherein the volume comprises a gas having the reduced pressure.

3. The cartridge (100) of any one of claims 1-2, further comprising a seal (118) coupled to the distal end portion (112) and covering the pierceable septum (116).

4. The cartridge (100) of any one of claims 1-3, further comprising a nut (124) detachably coupled to the proximal end portion (108) and securing the stopper (122) between the nut (124) and the plunger (120).

5. A syringe system for delivering an injectable drug to a subject, comprising:

   a cartridge (100) according to claim 1, the syringe system further comprising
   a vial (126) carrying the injectable drug;
   a transfer device configured to deliver the injectable drug from the vial (126) to the bore (104) of the cartridge (100); and
   a delivery needle (140) configured to be coupled to the distal end portion (112) of the cartridge (100) and deliver the injectable drug from the cartridge (100) to the subject,
   wherein the syringe system further comprises an actuating rod (142) configured to couple to the plunger (120).

6. The syringe system of claim 5, further comprising a distal seal (118) coupled to the distal end portion (112) and covering the pierceable septum (116).

7. The syringe system of any one of claim 5-6, wherein the transfer device comprises a transfer cannula (138) configured to pierce the pierceable septum (116).

8. The syringe system of any one of claims 5-7, wherein the transfer device comprises a flexible conduit (144).

9. A method of manufacturing a cartridge (100) according to claim 1, the cartridge (100) configured to receive an injectable drug, the method comprising:

   providing a body (106) comprising a distal opening (114), a proximal opening (110), and bore (104) extending between the distal opening (114) and the proximal opening (110);
   positioning a plunger (120) in the bore (104);
   coupling a stopper (122) to the proximal opening (110);
   reducing a pressure in the bore to less than atmospheric pressure; and
   sealing the distal opening (114),
   **characterised in that**:
   the stopper (122) is detachably coupled to the proximal opening (110); and
   the plunger (120) is configured to couple to an actuating rod (142) upon detachment of the stopper (122) from the body (106).

10. The method of claim 9, further comprising, prior to reducing the pressure in the bore (104), partially inserting a septum (116) into the distal opening (114) such that the bore (104) remains in fluid communication with an external environment of the body (106).

11. The method of claim 10, wherein:

sealing the distal opening (114) comprises further inserting the septum (116) into the distal opening (114) and covering the septum (116) with a seal; and/or

the method further comprises coupling a nut (124) to the body (106) and over the stopper (122).

12. The method of any one of claims 10-11, wherein reducing the pressure in the bore (104) comprises positioning the body (106) in a pressure reduction chamber (150).

13. The method of claim 12, further comprising, while the body (106) is positioned in the pressure reduction chamber (150), inserting a septum (116) into the distal opening (114) to inhibit fluid communication between the bore (104) and the pressure reduction chamber (150), and optionally further comprising removing the body (106) from the pressure reduction chamber (150), and wherein sealing the distal opening (114) comprises, after removing the body (106) from the pressure reduction chamber (150), covering the septum (116) with a seal.

14. A method of delivering a injectable drug from an internal chamber of a vial (126) to the internal bore (104) of a cartridge (100) according to claim 1, the internal bore (104) initially having a reduced pressure volume, the method comprising:

venting the internal chamber (130) of the vial (126) such that the injectable drug is exposed to atmospheric pressure;
coupling a transfer device to the vial (126);
coupling the transfer device to the cartridge (100), the transfer device thereby facilitating fluid communication between the internal chamber (130) of the vial (126) and the internal bore (104) of the cartridge (100); and
permitting the injectable drug to flow from the internal chamber (130) of the vial (126), through the transfer device, and into the internal bore (104) of the cartridge (100).

15. The method of claim 14, wherein:

coupling the transfer device to the vial (126) comprises piercing a septum (132) of the vial (126) with a needle of the transfer device;
coupling the transfer device to the cartridge (100) comprises piercing a septum (116) of the cartridge (100) with a needle of the transfer device; and/or
after permitting the injectable drug to flow into the internal bore (104) of the cartridge (100), the method further comprises:

decoupling the transfer device from the cartridge (100);
coupling a needle to the cartridge (100); and delivering the injectable drug to a subject via the needle.

**Patentansprüche**

1. Kartusche (100) zum Aufnehmen eines injizierbaren Arzneimittels, umfassend:
einen Körper (106), umfassend:

einen proximalen Endabschnitt (108), der eine proximale Öffnung (110) umfasst;
einen distalen Endabschnitt (112), der eine distale Öffnung (114) umfasst;
eine Bohrung (104), die die proximale Öffnung (110) und die distale Öffnung (114) koppelt;
ein durchstechbares Septum (116), das mit der distalen Öffnung (114) gekoppelt ist;
einen Kolben (120), der beweglich innerhalb der Bohrung (104) getragen wird;
ein Volumen, das in der Bohrung (104) zwischen dem durchstechbaren Septum (116) und dem Kolben (120) angeordnet ist, wobei das Volumen einen reduzierten Druck aufweist, wobei der reduzierte Druck geringer als der atmosphärische Druck ist;
**dadurch gekennzeichnet, dass**
ein Stopfen (122) lösbar mit der proximalen Öffnung (110) gekoppelt ist,
wobei der Kolben (120) konfiguriert ist, um beim Lösen des Stopfens (122) von dem Körper (106) mit einem Betätigungsstab (142) gekoppelt zu werden.

2. Kartusche (100) nach Anspruch 0, wobei das Volumen ein Gas umfasst, das den reduzierten Druck aufweist.

3. Kartusche (100) nach einem der Ansprüche 1 bis 2, ferner umfassend eine Dichtung (118), die mit dem distalen Endabschnitt (112) gekoppelt ist und das durchstechbare Septum (116) abdeckt.

4. Kartusche (100) nach einem der Ansprüche 1 bis 3, ferner umfassend eine Mutter (124), die lösbar mit dem proximalen Endabschnitt (108) gekoppelt ist und den Stopfen (122) zwischen der Mutter (124) und dem Kolben (120) sichert.

5. Spritzensystem zum Abgeben eines injizierbaren Arzneimittels an ein Subjekt, umfassend:

eine Kartusche (100) nach Anspruch 1, wobei das Spritzensystem ferner umfasst
eine Arzneiflasche (126), die das injizierbare Arzneimittel trägt;

eine Transfervorrichtung, die konfiguriert ist, um das injizierbare Arzneimittel aus der Arzneiflasche (126) in die Bohrung (104) der Kartusche (100) abzugeben; und

eine Abgabenadel (140), die konfiguriert ist, um mit dem distalen Endabschnitt (112) der Kartusche (100) gekoppelt zu werden und das injizierbare Arzneimittel von der Kartusche (100) an das Subjekt abzugeben,

wobei das Spritzensystem ferner einen Betätigungsstab (142) umfasst, der konfiguriert ist, um mit dem Kolben (120) gekoppelt zu werden.

6. Spritzensystem nach Anspruch 5, ferner umfassend eine distale Dichtung (118), die mit dem distalen Endabschnitt (112) gekoppelt ist und das durchstechbare Septum (116) abdeckt.

7. Spritzensystem nach einem der Ansprüche 5 bis 6, wobei die Transfervorrichtung eine Transferkanüle (138) umfasst, die konfiguriert ist, um das durchstechbare Septum (116) zu durchstechen.

8. Spritzensystem nach einem der Ansprüche 5 bis 7, wobei die Transfervorrichtung eine flexible Leitung (144) umfasst.

9. Verfahren zum Herstellen einer Kartusche (100) nach Anspruch 1, wobei die Kartusche (100) konfiguriert ist, um ein injizierbares Arzneimittel aufzunehmen, wobei das Verfahren umfasst:

Bereitstellen eines Körpers (106), der eine distale Öffnung (114), eine proximale Öffnung (110) und eine Bohrung (104) umfasst, die sich zwischen der distalen Öffnung (114) und der proximalen Öffnung (110) erstreckt;

Positionieren eines Kolbens (120) in der Bohrung (104);

Koppeln eines Stopfens (122) mit der proximalen Öffnung (110);

Reduzieren eines Drucks in der Bohrung auf weniger als den atmosphärischen Druck; und Abdichten der distalen Öffnung (114),

**dadurch gekennzeichnet, dass:**

der Stopfen (122) lösbar mit der proximalen Öffnung (110) gekoppelt ist; und

der Kolben (120) konfiguriert ist, um beim Lösen des Stopfens (122) von dem Körper (106) mit einem Betätigungsstab (142) gekoppelt zu werden.

10. Verfahren nach Anspruch 9, ferner umfassend, vor dem Reduzieren des Drucks in der Bohrung (104), das teilweise Einführen eines Septums (116) in die distale Öffnung (114), sodass die Bohrung (104) in Fluidverbindung mit einer externen Umgebung des Körpers (106) bleibt.

11. Verfahren nach Anspruch 10, wobei:

das Abdichten der distalen Öffnung (114) ferner das Einführen des Septums (116) in die distale Öffnung (114) und das Abdecken des Septums (116) mit einer Dichtung umfasst; und/oder das Verfahren ferner das Koppeln einer Mutter (124) mit dem Körper (106) und über den Stopfen (122) umfasst.

12. Verfahren nach einem der Ansprüche 10 bis 11, wobei das Reduzieren des Drucks in der Bohrung (104) das Positionieren des Körpers (106) in einer Druckreduzierungskammer (150) umfasst.

13. Verfahren nach Anspruch 12, ferner umfassend, während der Körper (106) in der Druckreduzierungskammer (150) positioniert ist, das Einführen eines Septums (116) in die distale Öffnung (114), um die Fluidverbindung zwischen der Bohrung (104) und der Druckreduzierungskammer (150) zu hemmen, und optional ferner umfassend das Entfernen des Körpers (106) aus der Druckreduzierungskammer (150), und wobei das Abdichten der distalen Öffnung (114) nach dem Entfernen des Körpers (106) aus der Druckreduzierungskammer (150) das Abdecken des Septums (116) mit einer Dichtung umfasst.

14. Verfahren zum Verabreichen eines injizierbaren Arzneimittels aus einer inneren Kammer einer Arzneiflasche (126) in die innere Bohrung (104) einer Kartusche (100) nach Anspruch 1, wobei die innere Bohrung (104) anfänglich ein reduziertes Druckvolumen aufweist, wobei das Verfahren umfasst:

Entlüften der inneren Kammer (130) der Arzneiflasche (126), sodass das injizierbare Arzneimittel atmosphärischem Druck ausgesetzt wird;

Koppeln einer Transfervorrichtung mit der Arzneiflasche (126);

Koppeln der Transfervorrichtung mit der Kartusche (100), wobei die Transfervorrichtung dadurch eine Fluidverbindung zwischen der inneren Kammer (130) der Arzneiflasche (126) und der inneren Bohrung (104) der Kartusche (100) erleichtert; und

Ermöglichen, dass das injizierbare Arzneimittel aus der inneren Kammer (130) der Arzneiflasche (126) durch die Transfervorrichtung und in die innere Bohrung (104) der Kartusche (100) fließt.

**15.** Verfahren nach Anspruch 14, wobei:

das Koppeln der Transfervorrichtung mit der Arzneiflasche (126) das Durchstechen eines Septums (132) der Arzneiflasche (126) mit einer Nadel der Transfervorrichtung umfasst; das Koppeln der Transfervorrichtung mit der Kartusche (100) das Durchstechen eines Septums (116) der Kartusche (100) mit einer Nadel der Transfervorrichtung umfasst; und/oder nach dem Ermöglichen, dass das injizierbare Arzneimittel in die innere Bohrung (104) der Kartusche (100) fließt, das Verfahren ferner umfasst:

Entkoppeln der Transfervorrichtung von der Kartusche (100); Koppeln einer Nadel mit der Kartusche (100); und Verabreichen des injizierbaren Arzneimittels an ein Subjekt über die Nadel.

**Revendications**

**1.** Cartouche (100) permettant de recevoir un médicament injectable, comprenant :
un corps (106) comprenant :

une partie d'extrémité proximale (108) comprenant une ouverture proximale (110) ; une partie d'extrémité distale (112) comprenant une ouverture distale (114) ; un alésage (104) accouplant l'ouverture proximale (110) et l'ouverture distale (114) ; un septum perçable (116) accouplé à l'ouverture distale (114) ; un piston (120) transporté de manière mobile au sein de l'alésage (104) ; un volume disposé dans l'alésage (104) entre le septum perçable (116) et le piston (120), le volume ayant une pression réduite, la pression réduite étant inférieure à la pression atmosphérique ; **caractérisée en ce que** un bouchon (122) est accouplé de manière amovible à l'ouverture proximale (110), dans laquelle le piston (120) est conçu pour s'accoupler à une tige d'actionnement (142) lors du détachement du bouchon (122) du corps (106).

**2.** Cartouche (100) selon la revendication 0, dans laquelle le volume comprend un gaz ayant la pression réduite.

**3.** Cartouche (100) selon l'une quelconque des revendications 1 à 2, comprenant en outre un joint (118) accouplé à la partie d'extrémité distale (112) et recouvrant le septum perçable (116).

**4.** Cartouche (100) selon l'une quelconque des revendications 1 à 3, comprenant en outre un écrou (124) accouplé de manière détachable à la partie d'extrémité proximale (108) et fixant le bouchon (122) entre l'écrou (124) et le piston (120).

**5.** Système de seringue permettant d'administrer un médicament injectable à un sujet, comprenant :

une cartouche (100) selon la revendication 1, le système de seringue comprenant en outre un flacon (126) contenant le médicament injectable ; un dispositif de transfert conçu pour administrer le médicament injectable du flacon (126) à l'alésage (104) de la cartouche (100) ; et une aiguille d'administration (140) conçue pour être accouplée à la partie d'extrémité distale (112) de la cartouche (100) et administrer le médicament injectable de la cartouche (100) au sujet, dans lequel le système de seringue comprend en outre une tige d'actionnement (142) conçue pour s'accoupler au piston (120).

**6.** Système de seringue selon la revendication 5, comprenant en outre un joint distal (118) accouplé à la partie d'extrémité distale (112) et recouvrant le septum perçable (116).

**7.** Système de seringue selon l'une quelconque des revendications 5 à 6, dans lequel le dispositif de transfert comprend une canule de transfert (138) conçue pour percer le septum perçable (116).

**8.** Système de seringue selon l'une quelconque des revendications 5 à 7, dans lequel le dispositif de transfert comprend un conduit flexible (144).

**9.** Procédé de fabrication d'une cartouche (100) selon la revendication 1,
la cartouche (100) étant conçue pour recevoir un médicament injectable, le procédé comprenant :

la fourniture d'un corps (106) comprenant une ouverture distale (114), une ouverture proximale (110), et un alésage (104) s'étendant entre l'ouverture distale (114) et l'ouverture proximale (110) ; le positionnement d'un piston (120) dans l'alésage (104) ; l'accouplement d'un bouchon (122) à l'ouverture proximale (110) ; la réduction d'une pression dans l'alésage à une pression inférieure à la pression atmosphérique ; et
le scellement de l'ouverture distale (114),
**caractérisé en ce que :**

le bouchon (122) est accouplé de manière amovible à l'ouverture proximale (110) ; et le piston (120) est conçu pour s'accoupler à une tige d'actionnement (142) lors du détachement du bouchon (122) du corps (106).

10. Procédé selon la revendication 9, comprenant en outre, avant de réduire la pression dans l'alésage (104), l'insertion partielle d'un septum (116) dans l'ouverture distale (114) de telle sorte que l'alésage (104) reste en communication fluidique avec un environnement externe du corps (106).

11. Procédé selon la revendication 10, dans lequel :

le scellement de l'ouverture distale (114) comprend en outre l'insertion du septum (116) dans l'ouverture distale (114) et le recouvrement du septum (116) avec un joint ; et/ou
le procédé comprend en outre l'accouplement d'un écrou (124) au corps (106) et par-dessus le bouchon (122).

12. Procédé selon l'une quelconque des revendications 10 à 11, dans lequel la réduction de la pression dans l'alésage (104) comprend le positionnement du corps (106) dans une chambre de réduction de pression (150).

13. Procédé selon la revendication 12, comprenant en outre, alors que le corps (106) est positionné dans la chambre de réduction de pression (150), l'insertion d'un septum (116) dans l'ouverture distale (114) pour inhiber la communication fluidique entre l'alésage (104) et la chambre de réduction de pression (150), et comprenant facultativement en outre le retrait du corps (106) de la chambre de réduction de pression (150), et dans lequel le scellement de l'ouverture distale (114) comprend, après le retrait du corps (106) de la chambre de réduction de pression (150), le recouvrement du septum (116) avec un joint.

14. Procédé d'administration d'un médicament injectable d'une chambre interne d'un flacon (126) à l'alésage interne (104) d'une cartouche (100) selon la revendication 1,
l'alésage interne (104) ayant initialement un volume de pression réduit, le procédé comprenant :

la mise à l'air libre de la chambre interne (130) du flacon (126) de telle sorte que le médicament injectable est exposé à la pression atmosphérique ;

l'accouplement d'un dispositif de transfert au flacon (126) ;
l'accouplement du dispositif de transfert à la cartouche (100), le dispositif de transfert permettant ainsi de faciliter la communication fluidique entre la chambre interne (130) du flacon (126) et l'alésage interne (104) de la cartouche (100) ; et
le fait de permettre au médicament injectable de s'écouler de la chambre interne (130) du flacon (126), à travers le dispositif de transfert, et dans l'alésage interne (104) de la cartouche (100).

15. Procédé selon la revendication 14, dans lequel :

l'accouplement du dispositif de transfert au flacon (126) comprend le perçage d'un septum (132) du flacon (126) avec une aiguille du dispositif de transfert ;
l'accouplement du dispositif de transfert à la cartouche (100) comprend le perçage d'un septum (116) de la cartouche (100) avec une aiguille du dispositif de transfert ; et/ou
après avoir permis au médicament injectable de s'écouler dans l'alésage interne (104) de la cartouche (100), le procédé comprend en outre :

le désaccouplement du dispositif de transfert de la cartouche (100) ;
l'accouplement d'une aiguille à la cartouche (100) ; et
l'administration du médicament injectable à un sujet par l'intermédiaire de l'aiguille.

## FIG. 1

**FIG. 2**

OBTAIN VIAL AND CARTRIDGE 〜200

↓

COUPLE TRANSFER DEVICE TO VIAL 〜202

↓

COUPLE CARTRIDGE TO TRANSFER DEVICE 〜204

↓

PERMIT DRUG TO FLOW FROM VIAL, THROUGH TRANSFER DEVICE, AND INTO CARTRIDGE 〜206

↓

UNCOUPLE VIAL, STOPPER, AND NUT FROM CARTRIDGE 〜208

↓

COUPLE NEEDLE AND ACTUATION ROD TO CARTRIDGE 〜210

FIG. 3

FIG. 4

FIG. 5

FIG. 6

FIG. 7

FIG. 8

126

128

134

136

100

128

122

124

FIG. 9

140

136

100

104

128

120

142

FIG. 10

300 → POSITION PLUNGER IN BORE OF CARTRIDGE

302 → COUPLE STOPPER AND NUT TO PROXIMAL END PORTION OF CARTRIDGE

304 → PARTIALLY INSERT SEPTUM INTO DISTAL OPENING OF CARTRIDGE

306 → POSITION CARTRIDGE IN PRESSURE REDUCTION CHAMBER

308 → REDUCE PRESSURE IN PRESSURE REDUCTION CHAMBER AND BORE OF CARTRIDGE

FULLY INSERT SEPTUM INTO DISTAL OPENING OF CARTRIDGE — 310

REMOVE CARTRIDGE FROM PRESSURE REDUCTION CHAMBER — 312

COUPLE SEAL TO DISTAL END PORTION OF CARTRIDGE — 314

FIG. 11

**FIG. 12**

**FIG. 13**

FIG. 14

FIG. 15

FIG. 16

**REFERENCES CITED IN THE DESCRIPTION**

**Patent documents cited in the description**

- US 63153420
- EP 1825878 A1
- US 2015013827 A1